# EUROPEAN PATENT APPLICATION

(11) **EP 4 001 421 A1**
(43) Date of publication of application: **25.05.2022**
(21) Application number: 20472015.5
(22) Date of filing: 16.11.2020
(51) Int. Cl.: C12P 7/66, C07C 46/10

(54) **METHOD FOR OBTAINING OIL EXTRACT OF MENAQUINONE-7 FROM FERMENTATION BROTH**

(71) Applicant: Endektovet Ltd, 4000 Plovdiv (BG)
(72) Inventor: Dorkov, Petar Dimitrov, 4550 Peshtera (BG)
(74) Representative: Kostadinova-Vulcheva, Zdravka D.

(57) **Abstract**

Our invention is related to a method for obtaining oil extract, containing menaquinone-7 from fermentation broth, characterized by thermal processing of the fermentation fluid at a temperature of 80 degrees, followed by direct continuous extraction c ethyl acetate and evaporation of the ethyl acetate for obtaining a highly concentrated extract containing MK-7. The direct extraction with ethyl acetate takes place as the ethyl acetate:fermentation broth ratio is between 1:1 and 1:3. The achievement of direct continuous extraction and complete regeneration of the organic solvent further improve the method effectiveness. The separation of the obtained organic phase from the aqueous phase is carried out through centrifuging or using a centrifugal separator. The concentration of the obtained extract is achieved by evaporation of the ethyl acetate in vacuum at a recovery of 90-98%.

The developed method for obtaining oil extract of menaquinone-7 from fermentation broth through continuous extraction ensures the effective isolation of the obtained vitamin K in a formulation convenient for use in the food and drink and pharmaceutical industries

## Description

### FIELD OF THE INVENTION

The invention is related to a method for obtaining oil extract of menaquinone-7 from fermentation BROTH, ensuring the efficient isolation of the obtained vitamin K in a formulation, convenient for use in the food and drink and pharmaceutical industries

### CURRENT STATE OF THE ART

It is known that menaquinones form part of the fat-soluble vitamin group of compounds, derivates of 2-methyl-1,4-naphthoquinone (3-). Menaquinones are usually jointly referred to as Vitamin K. Vitamin K2 is produced by bacteria (including intestinal bacteria) and is characterized by the number of isoprenoid (-CH2-C (CH) = CH-CH -) side chains. Menaquinones are usually marked as MK-n, where "M" means "menaquinone", "K" means "vitamin K", and "n" is the number of isoprenoid units in the side chain. MK-7 is the main representative in the Vitamin K2 group, as it is traditionally obtained through the fermentation of soybeans with a Bacillus subtilis natto strain. Recently, in order to increase productivity, MK-7 is being produced by deep-tank fermentation of the strain *Bacillus subtilis natto.*

MK-7 is a natural form of Vitamin K and thus Vitamin K2 participating in a number of metabolic processes, related to blood coagulation, bone metabolism and circulatory system. MK-7 has already been registered for use in the USA and the EU for the prevention of osteoporosis and some cardiovascular disorders. Currently MK-7 may be produced either by synthetic chemical methods or by fermentation, where *Bacillus subtilis* strains, such as as *Bacillus subtilis natto,* are usually used.

One of the unsolved issues in the production of MK-7 is its extraction from the fermentation fluid, due to the fact that its concentration is exceptionally law - in the range of micrograms and milligrams per litre.

In order to obtain a purified extract from fermentation fluid, several organic solvents are usually used, resulting in a number of issues, including process safety, multiple extraction stages, difficulties in solvents regeneration, residues of the solvents used in the endproduct and high production costs.

There is a traditional method of isolation of MK-7 (JP8073396) involving the use of a system of two solvents - isopropanol and hexane. The shortcoming of this method is that if used for the extraction from culture fluid, obtained through deep-tank cultivation, a large amount of solvents is used, and their re-generation is virtually impossible and an intermediary layer is formed at the phase boundary between the layers of waterisopropanol and hexane, which prevents the use of centrifugal separators. This means that the method is only applicable for the laboratory and semi-industrial production scale.

There is also another method, as detailed in WO 2014/131084 A1, where a lipid layer is used during the deep-tank cultivation, aimed at absorbing MK-7 during the fermentation process. After the end of the process, the lipid layer is separated and it contains a part of the obtained Vitamin K2. A disadvantage of this process is the low recovery, because it ensures no complete extraction of the vitamin obtained and the low concentration in the lipid layer, requiring further purification for producing an extract with high concentration.

There is another known process of MK-7 extraction from fermentation fluid with supercritical carbon dioxide, as described in 2329/MUM/2007 (Indian Application). This method is effective and environmentally-friendly, but it requires expensive equipment and is unsuitable for large-scale production.

Literature shows a mathematical model, allowing the design and use of spin-tests for determining the necessary equipment for the performance of continuous separation of non-mixing fluids. Merkel Tobias, Blättler Oliver and Königsson Staffan in 2018 detailed the use of a spin test for determining the technological equipment in chemical engineering and technologies. A laboratory test for the biomass separation in fullyhermetic disc centrifuge. It has been shown that the quality results of the laboratory screening are in conformance with the industrial-scale processes. The laboratory-scale experiments validated in pilot tests. There is a proven need of small amounts of samples, facilitating the quick and more efficient search for disc centrifuges with hermetic design that are suitable for use (41. 10.1002 /ceat.201800202). This approach is also used for solving technological problems by the best manufacturers of separation technologies, such as Alfa Faval (Sweden), Westfalia (Germany), Simon Nordestgsaer (Australia) etc.

The analysis of the known methods for obtaining or isolating vitamin K from the fermentation medium shows the need of new technical solutions for overcoming the said disadvantages.

The objective of the invention is to provide a method for obtaining an oil extract, containing menaquinone-7 from fermentation culture fluid, which would improve the extraction, increase the yield of menaquinone 7 and reduce the processes.

### TECHNICAL SUBSTANCE OF THE INVENTION:

The objective of the invention is achieved by a method for obtaining oil extract, containing menaquinone-7 from fermentation fluid, characterized by thermal processing of the fermentation fluid at a temperature of 80 degrees, followed by direct continuous extraction with ethyl acetate and evaporation of the ethyl acetate for obtaining a highly concentrated extract containing MK-7. The direct extraction using ethyl acetate with an ethyl acetate: fermentation fluid ratio between 1:1 and 1:3. The direct continuous extraction and complete regeneration of the organic solvent further improve the method effectiveness. The separation of the obtained organic phase from the aqueous phase is carried out through centrifuging or using a centrifugal separator. The concentration of the obtained extract is achieved by evaporation of the ethyl acetate in vacuum at a recovery of 90 - 98%

An advantage of the method, subject matter of the invention is that no use and further addition of coagulants is required. In the extraction processes based on the extraction of fat-soluble substances from an aqueous phase often use solvents, which do not mix with water, such as hexane, butyl acetate, etc. When the extraction from the fermentation fluid is performed, often a significant in volume intermediary layer between the organic and aqueous phases is formed, which impairs the extraction and consists of phospholipids, proteins and residues from the biomass of the producing strain. In such cases, various coagulants, such as aluminium trichloride, calcium trichloride, formaldehyde etc. are added. These coagulants may improve separation, but lead to further difficulties in purification, as these may even remain in the resulting product. The presence of an intermediary layer is undesirable because it also hinders the scaling of the process, by preventing the use of highly-efficient centrifugal separators. A combination of thermal processing and extractant ethyl acetate is achieved, resulting in the significant reduction of the intermediary layer without requiring the addition of coagulants. Another advantage of the method, subject matter of the invention, is the use of the solvent ethyl acetate. The use of ethyl acetate is compatible with the production of nutritional components. Ethyl acetate is characterized by low toxicity and permissible residues of up to 5000 ppm in the resulting end-products. Therefore, it is suitable for the extraction of active ingredients intended for use in food. Apart from that, easy regeneration of the solvent used is possible. The ethyl acetate is a low-boiling point solvent and is easily released from water-containing mixtures, through evaporation and condensation. Although the ethyl acetate vapours may contain up to several percent of water, after condensation and cooling this water forms a layer on the bottom of the container and may be easily separated. The regenerated ethyl acetate is ready for reuse.

### EXAMPLES FOR THE IMPLEMENTATION OF THE INVENTION

The various aspects of the invention are illustrated in the following examples.

All examples use fermentation broth, obtained through the following process: fermentation with the Bacillus subtilis natto strain in a 20-litre fermenter in a medium, containing corn starch, soy peptone, corn extract, soy tryptone. After 40 hours a fermentation broth is obtained, containing 35 g/l menaquinone-7.

### Example 1. Obtaining an oil concentrate containing menaquinone-7 through static extraction.

100ml culture broth, measured by a graduated cylinder, is placed in a 200ml glass with a magnet for magnetic agitator. The glass is placed on a magnetic agitator and the culture fluid is agitated. The culture broth is carefully heated at 80ºC. The obtained mixture is agitated for one hour at the said temperature. Upon completion of the thermal processing, the culture both is cooled down to room temperature.

50ml of the processed culture broth are placed into a 100ml separatory funnel and 50ml ethyl acetate are added. The funnel is shaken on a shaker for one hour.

Upon completion of the extraction process, the funnel is placed on a stand at rest in vertical position and the layering rate is monitored. After 2 hours at rest, good separation of the layers is observed in the test mixture, and the MK-7 content in the aqueous and organic phase are, as follows:
- Volume of the ethyl acetate layer - 51 ml
- content in the ethyl acetate layer 31 mg/L
- content in the aqueous phase <LOQ
- theoretical extraction yield - 90.3 %

The obtained ethyl-acetate extract is evaporated to dry matter in a calibrated flask on a rotational vacuum evaporator until dry oil concentrate is obtained.

### Example 2. Obtaining an oil concentrate containing menaquinone-7 through static extraction.

100ml culture broth, measured by a graduated cylinder, is transferred to a 200ml beaker. It is placed on a stirrer and the culture fluid is carefully heated at 80ºC with continuous stirring at 500 rpm for one hour. Upon completion of the thermal processing, the culture broth is cooled down to room temperature.

50ml of the processed culture fluid are placed in a 250ml separatory funnel and 150ml of ethyl acetate are added. The funnel is shaken on a shaker for one hour.

Upon completion of the extraction process, the funnel is placed on a stand at rest in vertical position and the layering rate is monitored. After 2 hours at rest, good separation of the layers is observed in the test mixture, as the MK-7 content in the aqueous and organic phase are, as follows:
- Volume of the ethyl acetate layer - 155 ml
- content in the ethyl acetate layer 10.4 mg/L
- content in the aqueous phase <LOQ
- theoretical extraction yield - 92.1 %

The obtained ethyl acetate extract is evaporated to dry matter in a calibrated flask on a rotational vacuum evaporator until dry oil concentrate is obtained.

### Example 3. Obtaining an oil concentrate containing menaquinone-7 through dynamic extraction.

100ml culture broth, measured by a graduated cylinder, is transferred to a 200ml beaker. It is placed on a stirrer and the culture fluid is carefully heated at 80ºC with continuous stirring at 500 rpm for one hour. Upon completion of the thermal processing, the culture fluid is cooled down to room temperature.

50ml of the processed culture fluid are placed into a 100ml separatory funnel and 50ml ethyl acetate are added. The funnel is shaken on a shaker for one hour.

Upon completion of the extraction process the entire content of the funnel is transferred to a centrifugal cuvette and centrifuged for 2 minutes at 3000 rpm. A clear division of the ethyl acetate layer and the water layer is achieved. The light ethyl acetate phase is carefully separated, using a pipette, as the MK-7 content in the aqueous and organic phase are, as follows:
- Volume of the ethyl acetate layer - 47 ml
- content in the ethyl acetate layer 34 mg/L
- content in the aqueous phase <LOQ
- theoretical extraction yield - 91.3 %

The obtained ethyl acetate extract is evaporated to dry matter in a calibrated flask on a rotational vacuum evaporator until dry oil concentrate is obtained.

### Example 4. Oil concentrate production process

The fermentation broth (with Menaquinone-7 content of 34 mg/L), upon completion of the fermentation process, is indirectly heated in the fermenter at 80ºC. Upon reaching the desired temperature, it is agitated for one hour at the said temperature. Upon completion of the thermal processing, the broth is cooled down to room temperature. 12,5L of the processed culture broth are transferred in two 32L graduated separatory containers and 12,5L of ethyl acetate are added. The mixture is agitated using a mechanical stirrer anchor stirring blades for one hour. After layering, the aqueous phase is extracted for a second time using the aforesaid amount of ethyl acetate.

After each extraction, the obtained mixture is left at rest until a clear boundary between the aqueous and organic phases is observed. The degree of separation and the appearance of both phases are visually assessed and a sample is submitted for analysing the content in the aqueous phase (slime).

The ethyl acetate extracts obtained from the extraction and layering processes, are concentrated on a rotary vacuum evaporator at 60ºC and vacuum of 240mbar to a volume of 1-2L.

The obtained ethyl acetate concentrate is subjected to hot decolouration at 60ºC as one percent active charcoal per volume is added (10-20g) for one hour. The processed concentrate is filtered under vacuum through a Büchner funnel, charged in a Büchner flask.

The obtained decoloured ethyl acetate concentrate is subjected to concentration to dry matter in a calibrated 1L distillation flask at 60ºC and vacuum of 240mbar.

The obtained dry residue in the calibrated one-litre flask is processed twice through distillation of 100ml 96% Ethanol, at 60ºC and vacuum of 175mbar. Upon complete distillation of the ethanol and cooling of the flask, the weight of the obtained dry residue in the calibrated flask is measured.

Refined sunflower oil is slowly added to the obtained dry residue in the calibrated one-litre flask, placed on laboratory scales, upon reaching a total final weight of 50g. The obtained mixture is heated at 60ºC until a clear homogenous oil solution is obtained. After cooling, the oil solution remains clear. The subsequent analysis established a 14.3 g/L or 715 mg menaquinone-7 content, which means an 84% total yield.

## Claims

1. Method for obtaining oil extract of menaquinone-7 from fermentation broth obtained through deep-tank cultivation of the *Bacillus subtilis natto* strain, **characterized by** the fact that it is based on a direct continuous extraction with ethyl acetate; temperature processing of the fermentation medium at temperatures in the range 78-82 degrees; separation of the organic phase obtained; concentration of the obtained extract through evaporation of the ethyl acetate;

2. A method, according to Claim 1, **characterized by** the fact that the ethyl acetate to fermentation fluid ratio is between 1:1 and 3:1;

3. A method, according to Claim 1, **characterized by** the fact that the separation of the obtained organic phase takes place in static conditions;

4. A method, according to Claim 1, **characterized by** the fact that the separation of the obtained organic phase from the aqueous phase takes place through centrifuging or using a centrifugal separator;

5. A method, according to Claim 1, **characterized by** the fact that the evaporation of the ethyl acetate takes place in vacuum conditions - from 175 mbar to 240 mbar
